# EUROPEAN PATENT SPECIFICATION

(11) **EP 2 154 144 B1**
(45) Date of publication and mention of the grant of the patent: **07.05.2014**
(21) Application number: 08161887.8
(22) Date of filing: 06.08.2008
(51) Int. Cl.: C07H 21/00, A61K 31/7088

(54) **Oligonucleotides and use thereof**
Oligonucleotide und ihre Verwendung
Oligonucléotides et leur utilisation

(43) Date of publication of application: 17.02.2010
(73) Proprietor: Changchun Huapu Biotechnology Co., Ltd., Changchun, Jilin 130021 (CN)
(72) Inventor: Wang, Liying, Changchun Jilin 130021 (CN); Hu, Dali, Changchun Jilin 130021 (CN); Sun, Ran, Changchun Jilin 130021 (CN); Yu, Yongli, Changchun Jilin 130021 (CN)
(74) Representative: Isarpatent

(56) References cited:
- WO-A-2006/028742
- HASHEM G M ET AL: "Hybrid oligomer duplexes formed with phosphorothioate DNAs: CD spectra and melting temperatures of S-DNA RNA hybrids are sequence-dependent but consistent with similar heteronomous conformation" BIOCHEMISTRY, AMERICAN CHEMICAL SOCIETY, EASTON, PA.; US, vol. 37, no. 1, 1 January 1998 (1998-01-01), pages 61-72, XP002970373 ISSN: 0006-2960
- HUNG SU-HWI ET AL: "Evidence from CD spectra that d(purine) cntdot r(pyrimidine) and r(purine) cntdot d(pyrimidine) hybrids are in different structural classes" NUCLEIC ACIDS RESEARCH, vol. 22, no. 20, 1994, pages 4326-4334, XP002500581 ISSN: 0305-1048
- DURAMAD OMAR ET AL: "Inhibitors of TLR-9 act on multiple cell subsets in mouse and man in vitro and prevent death in vivo from systemic inflammation" JOURNAL OF IMMUNOLOGY, AMERICAN ASSOCIATION OF IMMUNOLOGISTS, US, vol. 174, no. 9, 1 May 2005 (2005-05-01), pages 5193-5200, XP002380604 ISSN: 0022-1767
- BARRAT FRANCK J ET AL: "Nucleic acids of mammalian origin can act as endogenous ligands for toll-like receptors and may promote systemic lupus erythematosus" JOURNAL OF EXPERIMENTAL MEDICINE, ROCKEFELLER UNIVERSITY PRESS, JP, vol. 202, no. 8, 1 October 2005 (2005-10-01), pages 1131-1139, XP002380606 ISSN: 0022-1007
- ASHMAN ROBERT F ET AL: "Sequence requirements for oligodeoxyribonucleotide inhibitory activity" INTERNATIONAL IMMUNOLOGY, OXFORD UNIVERSITY PRESS, GB, vol. 17, no. 4, 1 April 2005 (2005-04-01), pages 411-420, XP002380605 ISSN: 0953-8178

## Description

### FIELD OF THE DISCLOSURE

The present invention relates to the use of an oligonucleotide for preparing a remedy for treating immune-mediated disorders.

### BACKGROUND ART

The present invention provides the use of an oligonucleotide which has a nucleotide sequence that fits the formula of (5'CCT3')n, for example the sequence of 5'-cctcctcctcctcctcctcctcct-3' (SEQ ID NO: 1) for preparing a remedy for treating an immune-mediated disorder.

The immune system protects human body from bacterial, parasitic, fungal, viral infections and from the growth of tumor cells. However, the immune response can sometimes be unwanted and cause immune-mediated disorder. The disorder includes autoimmune disease, graft rejection, hypersensitivity, diseases associated with the over-stimulation of host's immune system by microbes and Toll-like receptor (TLR)-mediated disease.

The autoimmune diseases results from an adaptive immune response and innate immune response or both against endogenous and/or exogenous antigens. Foreign substances, derived from bacteria, parasites, fungi or viruses, may mimic self-proteins and stimulate the immune system to launch responses to a self-cell and tissue, resulting in the diseases including but not limited to systemic lupus erythematosus (SLE) and rheumatoid arthritis. The graft rejection is a consequence of organ or tissue transplantation caused by the immune response in the transplant recipient (host) to the transplanted organ/tissue. When a subject is transplanted with grafts including kidney, pancrea, heart, lung, bone marrow, cornea and skin, the subject can launch an immune response (rejection) against the grafts. Hypersensitivity is an inappropriate immune response that has deleterious effects, resulting in significant tissue damage or even death. The hypersensitivity is divided into four types (e.g. Types I, II, III and IV. Disease associated with the over-stimulation of host's immune system by microbes is triggered by the infection of viruses such as flu viruses and other microbes. In the case of flu virus and Gram-negative bacterial infection, an excessive immune response to the invaders appears to be a fatal factor in patients. The response is characterized by the overproduction of cytokines. Studies of septic shock syndrome demonstrate that over production/aberrant production of cytokines can lead to rapid mortality due to cytokine-mediated lethal shock (Slifka MK, et al. J Mol Med. 2000; 78(2):74-80). Septic shock following gram-negative infection is a leading cause of mortality in critically ill patients. The exaggerated production of cytokines is known to contribute to sepsis characterized by cytokine-mediated lethal shock (Espat NJ, et al. J Surg Res. 1995 Jul; 59 (1):153-8). Multiple organ dysfunction syndromes (MODS) are a major cause of morbidity and mortality in severe sepsis and shock. Cytokine-mediated lethal shock resulted from over-production of host cytokines is considered a main mechanism leading to MODS (Wang H, et al. Am J Emerg Med. 2008 Jul; 26 (6):711-5). Toll-like receptor (TLR)-mediated disease is a disorder caused by the activation of Toll like receptors (TLRs). TLRs are a family of receptors that recognize microbe derived molecular structures (pathogen-associated molecular patterns or PAMPs). TLR expressing immune cells are activated upon binding of PAMPs. TLRs recognize a range of pathogen-derived products and activated. Lipopolysaccharide (LPS) of bacteria recognized by TLR4, lipotechoic acid and diacylated lipopeptides by TLR2-TLR6 dimmer, triacylated lipopeptides by TLR2-TLR1 dimmer, CpG containing oligonucleotide (CpG ODN) synthesized or derived from either viruses or bacteria by TLR9, bacterial flagellin by TLR5, zymosan by TLR2-TLR6 dimmer, F protein from respiratory syncytial virus (RSV) by TLR4, viral-derived double-stranded RNA (dsRNA) and poly I:C, a synthetic analog of dsRNA by TLR3; viral DNA by TLR9, single-stranded viral RNA (VSV and flu virus) by TLR7 and TLR8 (Foo Y. Liew, et al. Nature Reviews Immunology. Vol 5, June 2005, 446-458). In recent years, TLR activation has been connected to the pathogenesis of some of diseases including sepsis, dilated cardiomyopathy, diabetes, experimental autoimmune encephalomyelitis, systemic lupus erythematosus, atherosclerosis, asthma, chronic obstructive pulmonary disease and organ failure (Foo Y. Liew, et al. Nature Review Immunology, Vol 5, 2005, 446-458). Activation of TLR9 by self DNA play an important role in the development of autoimmune diseases such as SLE (Christensen SR, et al. Immunity 2006; 25:417-28) and rheumatoid arthritis (Leadbetter EA, et al. Nature 2002; 416:603-7; Boule MW, et al. J Exp Med 2004; 199:1631-40). Moreover, the elevated production of interferons (IFNs) resulted from TLR-9 activation has been reported to contribute to the develop of systemic lupus erythematosus (Barrat FJ, et al. J Exp Med 2005; 202:1131-9; Wellmann U, et al. Proc Natl Acad Sci USA 2005; 102:9258-63).

WO2006028742 relates to immunoregulatory polynucleotides comprising an immunoregulatory sequence (IRS). It is described that the oligonucleotides are applied in methods for inhibiting Toll-like receptor (TLR) 7/8 and/or 9 dependent innate immune responses. Two types of nucleotides are disclosed, namely oligonucleotides comprising the tetranucleotide sequence GGGG or comprising the nucleotide sequence TGCNm. The IRS of the oligonucleotides are disclosed to contain a 5'-G,C-3' sequence, at least one TGC trinucleotide sequence at or near the 5' end of the polynucleotide or a 5'-GGGG-3' sequence.

Duramad, Omar et al. disclose in "Inhibitors of TLR-9 act on multiple cell subsets in mouse and man in vitro and prevent death in vivo from systemic inflammation", THE JOURNAL OF IMMUNOLOGY, The American Association of Immunologists, Vol: 174, No: 9, Page(s): 5193 - 5200 that the optimal IRS to contain a GGGG motif.

Barrat Franck et al., "Nucleic acids of mammalian origin can act as endogenous ligands for toll-like receptors and may promote systemic lupus erythematosus", The Journal of Experimental Medicine, Rockefeller University Press, Vol: 202, No: 8, Page(s): 1131 - 1139, describe three series of oligonucleotide (ODN)-based inhibitors of Toll-like receptor (TLR) signaling. These ODN include sequences that inhibit both TLR7 and TLR 9.

In this invention, we disclose an oligonucleotide with a nucleotide sequence that fits the formula (5'CCT3')n for example that of 5'-cctcctcctcctcctcctcctcct-3' that inhibits proliferation of human PBMC activated by TLR9 agonist, inhibits interferon production from human PBMC induced by TLR9 agonist, HSV-1, flu virus and serum from SLE patients, and rescues the mice from cytokine induced shock. Therefore, this oligonucleotide is useful as a remedy for the treatment immune-mediated disorders.

The oligonuleotides of the invention inhibit TLR9 activation. It has been documented that TLR9 agonist activates both innate and adaptive immune response (Arthur M. Krieg. Nature Reviews Drug Discovery, Vol 5. June 2006, 471-484). CpG containing oligonucleotides (CpG ODN) is a TLR9 agonist [D.M. Klinman, Nat. Rev., Immunol. 4 (2004) 249- 258]. The oligonuleotides of the invention inhibits the proliferation and interferon production of human PBMC stimulated by CpG ODN, indicating that the oligonucleotide of the invention can be used as a remedy for the treatment of diseases related to TLR9 activation. Because TLR9 activation has been reported to contribute to the development of SLE (Barrat FJ, et al. J Exp Med 2005; 202:1131-9; Wellmann U, et al. Proc Natl Acad Sci USA 2005; 102:9258-63; Christensen SR, et al. Immunity 2006; 25:417-28) and rheumatoid arthritis (Leadbetter EA, et al. Nature 2002; 416:603-7; Boule MW, et al. J Exp Med 2004; 199:1631-40), the oligonucleotide of the invention can be used as a remedy for the treatment of SLE and rheumatoid arthritis by inhibiting the TLR9 activation.

The oligonuleotide of the invention inhibits the interferon production from human PBMC induced by TLR9 agonist, HSV-1, flu virus and serum from SLE patients. Because the elevated production of interferons has been reported to contribute to the development of SLE (Barrat FJ, et al. J Exp Med 2005; 202:1131-9; Wellmann U, et al. Proc Natl Acad Sci USA 2005; 102:9258-63), the oligonucleotide of the invention can be used as a remedy for the treatment of SLE by inhibiting IFN production.

The oligonuleotides of the invention inhibit the interferon production from human PBMC induced by flu virus (PR8). Since influenza virus has been documented to be able to activate TLR7 and TLR8 (Wang JP, et al. Blood. 2008 Jun 10. [Epub ahead of print]), the oligonucleotide of the invention can be used as a remedy for the treatment of Toll-like receptor (TLR)-mediated disease by inhibiting TLR7 or TLR8.

The oligonuleotides of the invention inhibit the interferon production from human PBMC induced by HSV-1. Since HSV-1 has been documented to activate TLR9 (Hubertus Hochrein et al. PNAS, 101, 11416-11421), the oligonucleotide of the invention can be used as a remedy for the treatment of Toll-like receptor (TLR)-mediated diseases including but not limited to SLE by inhibiting the activation of TLR9.

To study in vivo activity of the oligonucleotide of the invention, a mouse model of cytokine-mediated lethal shock was used. The mouse model was created by injecting CpG ODN into the D-galactosamine (D-Gal) presensentised mice. After being crated, the model mice died within 12 to 24 h. Analyses of plasma cytokines revealed over-production of tumor necrosis factor (TNF) alpha and interleukin-12 (IL-12) and gamma interferon (IFN-gamma) (Marshall AJ, et al. Infect Immun. 1998 Apr; 66(4):1325-33; Peter M, Bode K,et al. Immunology. 2008 Jan;123(1):118-28). By using the model, we demonstrate that the oligonucleotide of the invention can rescue mice from cytokine-mediated lethal shock. Because the cytokine-mediated lethal shock contributes to the septic shock (Slifka MK,et al. J Mol Med. 2000;78(2):74-80; Espat NJ, et al. J Surg Res. 1995 Jul;59(1):153-8) and multiple organ dysfunction syndromes (MODS) (Wang H, et al. Am J Emerg Mod. 2008 Jul;26(6):711-5), the oligonucleotide of the invention can be used as a remedy for the treatment of sepsis and MOGS by rescuing the host from cytokine-mediated lethal shock.

### DETAILED DESCRIPTION OF THE INVENTION

Unless otherwise noted, all terms in the invention have the same meaning as commonly understood by one of ordinary skill in the art to which this disclosure belongs. The singular terms "a," "an," and "the" include plural referents unless context indicates otherwise. Similarly, the word "or" is intended to include "and" unless the context indicates otherwise. In case of conflict, the present specification, including explanations of terms, will control. In addition, the materials, methods and examples are illustrative only and not intended to be limiting. Treat, treating or treatment shall have the same meaning without concerning the grammar. Similarly, prevent, preventing or prevention shall have the same meaning without concerning the grammar.

"Oligonucleotide": An oligonucleotide means multiple nucleotides (i.e. molecules comprising a sugar (e.g. deoxyribose) linked to a phosphate group and to an exchangeable organic base, which is either a substituted pyrimidine (Py) (e.g., cytosine (C), thymine (T)) or a substituted purine (Pu) (e.g., adenine (A) or guanine (G)). The term oligonucleotide as used herein refers to oligodeoxyribonucleotide (ODN). The oligonucleotide can be obtained from existing nucleic acid sources (e.g., genomic or cDNA), but are preferably synthetic. The oligonucleotide of the invention can be synthesized by a variety of automated nucleic acid synthesizers available in the market. These oligonucleotides are referred to as synthetic oligonucleotides.

"Chemical modification": The oligonucleotide disclosed in the invention can encompass various chemical modifications, in comparison to natural DNA, involving a phosphodiester internucleoside bridge, a ribose unit and/or a natural nucleoside base (adenine, guanine, cytosine, thymine). The modifications can occur either during or after synthesis of the oligonucleotide. During the synthesis, modified bases can be incorporated internally or on its end. After the synthesis, the modification can be carried out using the active groups (via an amino modifier, via the 3' or 5' hydroxyl groups, or via the phosphate group). The skilled person knows examples of chemical modifications. An oligonucleotide according to the invention may have one or more modifications, wherein each modification is located at a particular phosphodiester internucleoside bridge and/or at a particular ribose unit and/or at a particular natural nucleoside base position in comparison to an oligonucleotide of the same sequence, which is composed of natural DNA. The chemical modification includes "back bone modification" of the oligonucleotide of the invention. As used herein, the modified back bone of the oligonucleotide of the invention includes, but not limited to the "phosphorothioate backbone" that refers to a stabilized sugar phosphate backbone of a nucleic acid molecule in which a non-bridging phosphate oxygen is replaced by sulfur at least one internucleotide linkage. In one embodiment a non-bridging phosphate oxygen is replaced by sulfur at each and every internucleotide linkage. Other back bone modifications denote the modification with nonionic DNA analogs, such as alkyl-and aryl-phophonates (in which the charged phosphonate oxygen is replaced by an alkyl or aryl group), phophodiester and alkylphosphotriesters, in which the charged oxygen moiety is alkylated. In other examples, the oligonucleotide can be is a phosphorothioate/phosphodiester chimera. The chemical modification also includes the base substitutions of the oligonucleotide disclosed in the invention. The substituted purines and pyrimidines can be C-5 propyne pyrimidine and 7-deaza-7-substituted purine. The substituted purines and pyrimidines include but are not limited to adenine, cytosine, guanine, and thymine, and other naturally and non-naturally occurring nucleobases. The chemical modification of the oligonucleotide of the invention further includes the modification of the bases of the oligonucleotide. A modified base is any base which is chemically distinct from the naturally occurring bases typically found in DNA such as T, C, G and A, but which share basic chemical structures with these naturally occurring bases. The oligonucleotide of the invention can be modified by using cytidine derivatives. The term "cytidine derivative" refers to a cytidine-like nucleotide (excluding cytidine) and the term "thymidine derivative" refers to a thymidine-like nucleotide (excluding thymidine). In addition, the oligonucleotides of the invention can be chemically modified by linking a diol, such as tetraethyleneglycol or hexaethyleneglycol, at either or both termini of the oligonuleotide.

"Immune-mediated disorder": An immune-mediated disorder is a disease caused by an unwanted immune response in a subject. The disorder includes autoimmune disease, graft rejection, hypersensitivity, diseases associated with the over-stimulation of host's immune system by microbes and diseases associated with TLR activation. The oligonucleotide disclosed in the invention can be used as a remedy to treat the immune-mediated disorder.

"Immune response": A response of a cells of the immune system, such as a B cell, T cell, natural killer cell, dendritic cell, neutraphil and macrophage to a stimulus. The response includes innate immune response and adaptive (specific or acquired) immune response. The adaptive (specific or acquired) immune response includes humoral immune response and cellular immune response.

"Prevent or treat immune-mediated disorder": As used herein, prevent refers to prevent the full development of an immune-mediated disorder in a subject; treat refer a therapeutic intervene in a subject so as to ameliorate a sign or symptom of, halt the progression of, or eliminate pathological condition of the immune-mediated disorder.

"Subject": As used herein, a subject refers to a human or non-human vertebrate. Non-human vertebrates are non-human primates, livestock animals and companion animals. The oligonucleotide of the invention can be administered to prevent or/and treat immune-mediated disorder in a subject.

"Autoimmune diseases": The term "autoimmune disease" refers to a disease caused by a breakdown of self-tolerance such that the adaptive and innate immune system responds to self antigens and mediates cell and tissue damage. Autoimmune diseases are frequently characterized by means of their involvement of single organ or single cell-types or involvement of multiple organs or tissue systems. Autoimmune diseases have also been referred to as "collagen," or "collagen-vascular" or "connective tissue" diseases. Autoimmune disorders are frequently associated with hypersensitivity reactions. The oligonucleotides of the invention can be useful for treating and/or preventing various types of autoimmune diseases. Specific, non-limiting examples of autoimmune disorders are systemic lupus erythematosus, insulin-dependent (type I) diabetes mellitus, inflammatory arthritis, rheumatoid arthritis, multiple sclerosis, autoimmune hepatitis, chronic aggressive hepatitis, autoimmune hemolytic anemia, autoimmune thrombocytopenia, autoimmune atrophic gastritis of pernicious anemia, autoimmune encephalomyelitis, autoimmune orchitis, acquired hemophilia, ankylosing spondylitis, antiphospholipid syndrome, Beh.cedilla.et's syndrome, cardiomyopathy, chronic inflammatory demyelinating polyneuropathy, cicatricial pemphigoid, cold agglutinin disease, polymyositisdermatomyositis, discoid lupus, sympathetic ophthalmia, essential mixed cryoglobulinemia, fibromyalgia, fibromyositis, Guillain-Barr syndrome, idiopathic pulmonary fibrosis, idiopathic thrombocytopenic purpura, IgA nephropathy, juvenile arthritis, systemic sclerosis, polyarteritis nodosa, polychondritis, dermatomyositis, primary agammaglobulinemia, primary biliary cirrhosis, hyperimmunoglobulin E, progressive systemic sclerosis, psoriasis, Reiter's syndrome, sarcoidosis, stiff-man syndrome, uveitis, vasculitis, vitiligo, Hashimoto's thyroiditis, Goopasture's disease, pernicious anemia, Addison's disease, dermatomyositis, Sjogren's syndrome, dermatomyositis, myasthenia gravis, Grave's disease, uveitis, allergic encephalomyelitis, glomerulonephritis, and the like (N Engl J Med, Vol. 345, No. 5, August 2, 2001, p340-350). DNA or RNA released from DNA- or RNA-containing microbes could stimulate the production of autoantibody specific to self RNA- or DNA-containing complexes and consequently led to an autoimmune disease, including but not limited to SLE.

"Hypersensitivity": A hypersensitivity is referred to the disorders wherein tissue injury occurs as a result of a humoral or cell-mediated response to antigens of endogenous or exogenous origin and has been classified into four types. Type I hypersensitivity (frequently referred to as anaphylactic, immediate-type, atopic, reagenic, IgE-mediated hypersensitivity reactions or allergy) generally result from the release of pharmacologically active substances such as histamine, slow-reacting substance of anaphylaxis (SRS-A), and eosinophilic chemotactic factor (ECF) form IgE-sensitized basophils and mast cells after contact with a specific exogenous antigen. Type I hypersensitivity includes, but not limited to, allergic extrinsic asthma, seasonal allergic rhinitis and systemic anaphylaxis. Type II hypersensitivity (also referred to as cytotoxic, cytolytic complement-dependent or cell-stimulating hypersensitivity reaction) results when antibody reacts with antigenic components of cells or tissue elements or with an antigen or hapten, which has become intimately coupled to cells or tissue. Type II hypersensitivity includes, but not limited to, autoimmune hemolytic anemia, erythroblastosis fetalis and Goodpasture's disease. Type III hypersensitivity (also referred to as toxic complex, soluble complex, or immune complex hypersensitivity reactions) results from the deposition of soluble circulating antigen-antibody complexes in vessels or in tissues, with accompanying acute inflammatory reactions at the site of immune complex deposition. Type III hypersensitivity includes, but not limited to, Arthurs reaction, serum sickness, systemic lupus erythematosis, and certain types of glomerulonephritis. Type IV hypersensitivivity (frequently called cellular, cell-mediated, delayed, or tuberculin-type hypersensitivity reactions) are caused by sensitized T-lymphocytes which result from contact with a specific antigen. Type IV hypersensitivity includes, but not limited to, contact dermatitis and allograft rejection (Richard A. et al. Immunology, Fifth Edition, 2003, W.H. FREEMAN AND COMPANY).

"Diseases associated with the over-stimulation of host's immune system by microbes": Microbe invasion, if severe, sometimes can cause systemic inflammatory response in a subject, leading to diseases associated with the over-stimulation of host's immune system by microbes. The events in the development of the diseases, such as in the case of influenza A (H5N1) or bacterial infection, include the significantly elevated blood levels of tumor necrosis factor (TNF-α), interleukin 1 (IL-1), IL-6, IL-12, interferon α (IFN-α), interferon β (IFN-β), interferon y, chemokines interferon-inducible protein 10, monocyte chemoattractant protein 1, interleukin-8, interleukin-1β, and monocyte chemoattractant protein 1. Such responses can result in cytokine-mediated lethal shock that is responsible in part for the sepsis, ARDS, and multiorgan failure observed in many patients (The Writing Committee of the World Health Organization (WHO) Consultation on Human Influenza A/H5. Avian Influenza A (H5N1) Infection in Humans. N Eng1 J Med 2005; 353:1374-85). The significantly elevated blood level of cytokines followed microbe infection is termed by hypercytokinemia (hypercytokinaemia) or a cytokine storm. The research suggested that patients who contract bird flu or SARS may need drugs that suppress the immune response in addition to anti-viral drugs. The oligonucleotide of the invention can be used to treat and/or prevent the diseases associated with the stimulation of host's immune system by microbes in a subject. The microbes causing the diseases includes, but not limited to, viruses, bacteria, fungi, parasites and etiological agents of Spongiform encephalopathies. The virus that cause the diseases associated with the over-stimulation of host's immune system by microbes include: SARS CoV, influenza viruses, avian flu virus H1V-1, polio viruses, hepatitis A virus; enteroviruses, human Coxsackie's viruses, rhinoviruses, echoviruses, equine encephalitis viruses, rubella viruses, dengue viruses, encephalitis viruses, yellow fever viruses, corona viruses, vesicular stomatitis viruses, rabies viruses, Ebola viruses, parainfluenza viruses, mumps virus, measles virus, respiratory syncytial virus, influenza viruses, Hantan viruses, bunga viruses, phleboviruses, Nairo viruses, hemorrhagic fever viruses; reoviruses, orbiviurses and rotaviruses, Hepatitis B virus, parvoviruses, papilloma viruses, polyoma viruses, adenoviruses, herpes simplex virus (HSV) 1 and HSV-2, varicella zoster virus, cytomegalovirus (CMV), herpes viruses, variola viruses, vaccinia viruses, pox viruses, African swine fever virus, the etiological agents of Spongiform encephalopathies, delta hepatitis virus, Hepatitis C virus, foot and mouth disease virus and avian flu virus. The bacteria that can cause the diseases associated with the over-stimulation of host's immune system by microbes include: Helicobacter pyloris, Borelia burgdorferi, Legionella pneumophilia, Mycobacteria sps (such as. M. tuberculosis, M. avium, M. E intracellulare, M. kansaii, M. gordonae), Staphylococcus aureus, Neisseria gonorrhoeae, Neisseria meningitidis, Listeria monocytogenes, Group A Streptococcus, Group B Streptococcus, Streptococcus, Streptococcusfaecalis, Streptococcus bovis, Streptococcus (anaerobic sps.), Streptococcus pneumoniae, pathogenic Campylobacter sp., Enterococcus sp., Haemophilus influenzae, Bacillus antracis, corynebacterium diphtheriae, corynebacterium sp., Erysipelothrix rhusiopathiae, Clostridium perfringers, Clostridium tetani, Enterobacter aerogeytes, Klebsiella pneumoniae, Pasturella multocida, Bacteroides sp., Fusobacterium nucleatum, Streptobacillus moniliformis, Treponema pallidium, Treponema pertenue, Leptospira, and Actinomyces israelli. The fungi that can cause the diseases associated with the over-stimulation of host's immune system by microbes include, but not limited to, Cryptococcus neoformans, Histoplasma capsulatum, Coccidioides immitis, Blastomyces dermatitidis, Chlamydia trachomatis, Candida albicans. The parasites that can cause the diseases associated with the over-stimulation of host's immune system by microbes include: Plasmodium falciparum and Toxoplasma gondii.

"Graft rejection": The graft rejection is an immune-mediated disorder caused by organ or tissue transplantation, Transplantation means the transfer of transplants (grafts) from a donor to a recipient. Grafts are the living cells, tissues, or organs transplanted from a donor to a recipient. An autograft is the a graft transferred of one's own tissue from one location to another; a syngeneic graft (isograft) is a graft between identical twins; an allogeneic graft (homograft) is a graft between genetically dissimilar members of the same species; and a xenogeneic graft (heterograft) is a transplant between members of different species. When a subject is the recipient of an allogeneic graft or a xenogeneic graft, the body can produce an immune response against the donor tissue. In this situation, there is a clear need to suppress the immune response, in order to avoid rejection of the graft (Richard A. et al. Immunology, Fifth Edition, 2003, W.H. FREEMAN AND COMPANY). The oligonucleotides of the present invention are useful when administered for the prevention of the graft rejection. Examples of the grafts are heart, kidney, liver, medulla ossium, skin, cornea, lung, pancreas, intestinum tenue, limb, muscle, nervus, duodenum, small-bowel, pancreatic-islet-cell, and the like. In some case, the recipient may be an animals as defined in "subject"ofthe invention.

"Toll-like receptor (TLR)-mediated diseases": A Toll-like receptor (TLR)-mediated disease means an immune mediated disorder related to the activation of members of the TLR family. The disease includes, but not limited to, the diseases include but not limited to, sepsis associated with the activation of TLR4 by lipopolysaccharide (LPS), dilated cardiomyopathy associated with the activation of TLR2, 3, 4, 9, diabetes associated with the activation of TLR2,3,4,9, experimental autoimmune encephalomyelitis associated with the activation of TLR3, systemic lupus erythematosus associated with the activation of TLR9, atherosclerosis associated with the activation of TLR4, asthma associated with the activation of TLR4 by LPS, chronic obstructive pulmonary disease associated with the activation of TLR4, EAE associated with the activation of TLR4 and organ failure associated with the activation of TLR4 (Foo Y et al. Nature Review Immunology, Vol 5, 2005, 446-458). CpG-containing DNA (a TLR9 agonist) derived from a nucleic acid-containing infectious agent could be identified from SLE serum that induces an efficient immune response dominated by IFN-α secretion that is thought to contribute the development of SLE. The oligonucleotides of the present invention can be administered for treating and/or prevent the Toll-like receptor (TLR)-mediated diseases including but not limited to SLE in a subject.

"CpG ODN": It has been documented that TLR9 agonist activates both innate and adaptive immune response (Arthur M. Krieg. Nature Reviews Drug Discovery, Vol 5. June 2006, 471-484). CpG containing oligonucleotides (CpG ODN) is a TLR9 agonist [D.M. Klinman, Nat. Rev., Immunol. 4 (2004) 249- 258]. Based on the functional characteristics, CpG ODNs are divided into three types (Tomoki Ito, et al. Blood, 2006, Vol 107, Num 6: 2423-2431). A-type CpG ODN activates human plasmacytoid dendritic cells (pDCs) to produce large amount of type I interferon (IFN-a/β) and strongly activates natural killer cells (NK cells). B-type CpG ODN primarily activates B cells, resulting in their proliferation and antibody secretion. C-type CpG ODN shares the activities of both A- and B-type CpG ODN. As a TLR9 agonist, CpG ODN such as CpG 2216 or CpG 2006 or CpG C274 can be endocytosed into a cellular compartment where they are exposed to and activate TLR9. In pDC, TLR9 activation initiate a rapid innate immune response that is characterized by the secretion of pro-inflammatory cytokines [IL-6, tumor-necrosis factor-α (TNFα)], the secretion of type I interferon (IFN) and the secretion of secretion of IFN-inducible chemokines. Through both IFN-dependent and IFN-independent pathways, innate immune cells including natural killer (NK) cells, monocytes and neutrophils are secondarily activated by the pDC. B cells activated through TLR9 have a greatly increased sensitivity to antigen stimulation and efficiently differentiate into antibody-secreting cells, and therefore contributing to the adaptive immune response, especially humoral immune response. pDC activated through TLR9 secrete IFNα, which drives the migration and clustering of pDC to lymph nodes and other secondary lymphoid tissues where the pDC activates naive and memory T cells, assists the cross-presentation of soluble protein antigens to CD8+ cytotoxic T lymphocyte (CTL) and promotes strong TH1 biased cellular CD4 and CD8 T-cell responses. Based on the above mentioned findings, it is obvious that the agents that antagonize the activity of CpG ODN can be used to treat or prevent the immune-mediated disorder by inhibiting both innate and adaptive immune response.

"Pharmaceutically acceptable carrier": A pharmaceutically acceptable carrier denotes one or more solid or liquid filler, diluents or encapsulating substances that are suitable for administering the oligonucleotide of the invention to a subject. The carrier can be organic, inorganic, natural or synthetic. The carrier includes any and all solutions, diluents, solvents, dispersion media, liposome, emulsions, coatings, antibacterial and anti-fungal agents, isotonic and absorption delaying agents, and any other carrier suitable for administering the oligonucleotide of the invention and their use is well known in the art. The pharmaceutically acceptable carriers are selected depending on the particular mode of administration of the oligonucleotide. The parenteral formulations usually comprise injectable fluids that include pharmaceutically and physiologically acceptable fluids such as water, physiological saline, balanced salt solutions, aqueous dextrose, glycerol or the like as a vehicle. For solid compositions (e. g., powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

"Therapeutically effective amount": In order to treat or prevent an immune-mediated disorder, a therapeutically effective amount of an oligonucleotide of the invention is administered to a subject. The "therapeutically effective amount" of one of the oligonucleotides means a sufficient amount of the oligonucleotide used to achieve a desired result of treating or preventing an immune-mediated disorder in a subject. The oligonucleotides of the present invention may be employed in pure form or in pharmaceutically acceptable carriers. Alternatively, the oligonucleotides may be administered as pharmaceutical compositions. The "amount" in the invention shall refer to a dose. The dose can be determined by standard techniques well known to those skilled in the art and can vary depending the factors including, but not limited to the size or/and overall health of the subject or the severity of the disease. Introduction of the oligonucleotide of the invention can be carried out as a single treatment or over a series of treatments. Subject doses of the oligonucleotide of the invention for the administration range from about 1 µg to 100 mg per administration. However, doses for the treatment of immune-mediated disorder may be used in a range of 10 to 1,000 times higher than the doses described above. The more preferable doses can be adjusted to provide the optimum therapeutic effect by those skilled in the art, for example, by the attending physician within the scope of sound medical judgment.

"Route of administration": For clinical use, the oligonucleotides of the invention can be administered alone or formulated in a pharmaceutical composition via any suitable route of administration that is effective to achieve the desired therapeutic result. The "route" of administering the oligonucleotide of the invention shall mean the enteral, parenteral and topical administration or inhalation. The enteral routes of administration of the oligonucleotide of the invention include oral, gastric, intestinal, and rectal. The parenteral route includes intravenous, intraperitoneal, intramuscular, intrathecal, subcutaneous, local injection, vaginal, topical, nasal, mucosal, and pulmonary administration. The topical route of administration of the oligonucleotide of the invention denotes the application of the oligonucleotide externally to the epidermis, to the buccal cavity and into the ear, eye and nose.

"Pharmaceutical composition": A pharmaceutical composition shall mean the composition comprising an therapeutically effective amount of the oligonucleotide of the invention with or without a pharmaceuticaliy acceptable carrier. The pharmaceutical compositions can comprise one or more oligonucleotides of the invention. The composition includes but not limited to aqueous or saline solutions, particles, aerosols, pellets, granules, powders, tablets, coated tablets, (micro) capsules, suppositories, syrups, emulsions, suspensions, creams, drops and other pharmaceutical compositions suitable for use in a variety of drug delivery systems. The compositions may be administered parenterally, orally, rectally, intravaginally, intraperitoneally, topically (in a dosage form as powders, ointments, gels, drops or transdermal patch), bucally, or as an oral or nasal spray. In all cases, the composition must be sterile and stable under the conditions of manufacture and storage and preserved against the microbial contamination. Pharmaceutical compositions of this invention for parenteral injection comprise pharmaceutically-acceptable sterile aqueous or nonaqueous solutions, dispersions, suspensions or emulsions, as well as sterile powders for reconstitution into sterile injectable solutions or dispersions just prior to use. The oligonucleotide of the invention can be suspended in an aqueous carrier, for example, in an isotonic buffer solution at a pH of about 3.0 to about 8.0, preferably at a pH of about 3.5 to about 7.4, 3.5 to 6.0, or 3.5 to about 5.0. The buffer solution includes sodium citrate-citric acid and sodium phosphate-phosphoric acid, and sodium acetate-acetic acid buffers. For oral administration, the composition will be formulated with edible carriers to form powders tablets, pills, dragees, capsules, liquids, gels, syrups, slurries, suspensions and the like. For solid compositions, conventional non-toxic solid carriers can include pharmaceutical grades of mannitol, lactose, starch, or magnesium stearate. For buccal administration, the composition will be tablets or lozenges in conventional manner. For inhalation, the composition will be an aerosol spray from pressurized packs or a nebulizer or a dry powder and can be selected by one of skill in the art. In some cases, in order to prolong the effect of the oligonucleotide of the invention, the oligonucleotides of the invention are also suitably administered by sustained-release systems. The oligonucleotide of the invention can be used in a liquid suspension of crystalline or amorphous material with poor water solubility to slow the releasing of the oligonucleotide. Alternatively, delayed releasing of a parenterally administered drug form of the oligonucleotide is accomplished by dissolving or suspending the oligonucleotide in hydrophobic materials (such as an acceptable oil vehicle). Injectable depot forms are made by entrapping the oligonucleotide in liposomes or microemulsions or other biodegradable semi-permeable polymer matrices such as polylactide-polyglycolide, poly (orthoesters) and poly (anhydrides).

"Active ingredients": The oligonucleotides of the invention can be used alone, in combination with themselves, in a pharmaceutically acceptable carrier, in combination with one or more additional active ingredients. The administration of the oligonucleotide of the invention and additional active ingredients can be sequential or simultaneous. The active ingredients include non-steroidal anti-inflammatory agents, steroids, nonspecific immunosuppressive agent, biological response modifier, chemical compound, small molecule, nucleic acid molecule and TLR antagonists. The active ingredients also denote the agents that suppress the immune activation by antagonizing chemochines, by inducing the generation of regulatory T cells (CD4+CD25+ T cells), by inhibiting a complement, matrix metalloproteases and nitric oxide synthase, by blocking costimulatory factors and by inhibiting the signaling cascades in the immune cells. The non-steroidal anti-inflammatory agents include, but unlimited to, diclofenac, diflunisal, etodolac, flurbiprofen, ibuprofen, indomethacin, ketoprofen, ketorolac, nabumetone, naproxen, oxaprozin, piroxicam, sulindac, tohnetin, celecoxib and rofecoxib. The steroids include, but unlimited to, cortisone, dexamethasone, hydrocortisone, methylprednisolone, prednisolone, prednisone, and triamcinolone. A nonspecific immunosuppressive agent means the agent used to prevent the development of immune-mediated disorder. The nonspecific immunosuppressive agents include but not limited to cyclophosphamide, cyclosporine, methotrexate, steroids, FK506, tacrolimus, mycophenolic acid and sirolimus. The biological response modifier includes a recombinant interleukin-1-receptor antagonist (Kineret or anakima), a soluble p75 TNF-a receptor-IgG1 fusion protein (etanercept or Enbrel), or a monoclonal antibody against TNF-a (infliximab or RemicadeX). The agents also include Interferon beta-la, interleukin-10 and TGFβ.

"Delivery vehicle": The oligonucleotides of the invention can be administered in/with a delivery vehicle or in a form linked with a vehicle. The vehicle includes, but not limited to, sterol (e.g., cholesterol), cochleates, emulsomes, ISCOMs; a lipid (e.g., a cationic lipid, anionic lipid), liposomes; ethylene glycol (PEG); live bacterial vectors (e.g., Salmonella, Escherichia coli, bacillus Calmette-Gurin, Shigella, Lactobacillus), live viral vectors (e.g., Vaccinia, adenovirus, Herpes simplex), virosomes, virus-like particles, microspheres, nucleic acid vaccines, polymers (e.g., carboxymethylcellulose, chitosan), polymer rings and a targeting agent that recognizes target cell by specific receptors.

"Pegylation": Pegylation is the process of covalent attachment of poly (ethylene glycol) polymer chains to another molecule, normally a drug or therapeutic protein. Pegylation is routinely achieved by incubation of a reactive derivative of PEG with the target agent. The pegylated agent can "mask" the agent from the host's immune system, increase the hydrodynamic size of the agent which prolongs its circulatory time. The oligonucleotides of the invention can be pegylated.

### BRIEF SUMMARY OF SPECIFIC EMBODIMENTS

The present invention is directed to the use of an oligonucleotide according to claim 1.

In a first embodiment, the present invention provides an oligonucleotide with a nucleotide sequence of 5'-cctcctcctcctcctcctcctcct-3' (SEQ ID NO: 1) and the oligonucleotides that fit the formula of (5' CCT 3') n.

The present invention provides a remedy for treating immune-mediated disorder using the oligonucleotide of the invention. The immune-mediated disorder is systemic lupus erythematosus, sepsis, multiple organ dysfunction syndrome or psoriasis.

Herein it is described a remedy for treating immune-mediated disorder using the oligonucleotides of the invention by inhibiting the TLR activation and IFN production induced by DNA virus, RNA virus, the serum from SLE patients, and by rescuing a subject from cytokine-mediated lethal shock.

In a further embodiment, the present invention provides a remedy for treating immune-mediated disorder by administering the oligonucleotide of the invention alone or with a pharmaceutically acceptable carrier to a subject through the route of enteral, parenteral and topical administration or inhalation.

Further, the present invention describes a composition comprising therapeutically effective amount of the oligonucleotides of the invention for the treatment of immune-mediated disorder.

In another embodiment, the present invention provides a remedy for the treatment of immune-mediated disorder by administering the oligonucleotides of the invention alone or in combination with additional active ingredients.

In another embodiment, the present invention provides a remedy for the treatment of immune-mediated disorder by administering the oligonucleotide of the invention in delivery vehicles.

Thereby, in a aspect, the present invention provides the oligonucleotide includes a sequence that fits the formula of (5' CCT 3') n, wherein 5' CCT 3' is a repeat unit and n is an integer from 2 to 50, preferably, it is 5'-cctcctcctcctcctcctcctcct-3' (SEQ ID NO: 1).

In a preferable embodiment, the phosphate backbone of the oligonucleotide can be partly or completely phosphorothioate-modified, or unmodified.

In another preferable embodiment, the oligonucleotide can be developed into their derivatives by adding in one or several nucleotides to each end of the oligonucleotide and by changing one or several bases in the oligonucleotide.

In another preferable embodiment, the oligonucleotide constitutes a part of other DNA molecules, plasmid or viral vectors.

In an even more preferable embodiment, the oligonucleotide can undergo chemical modification.

In another aspect, the present invention provides use of above oligonuleotide for preparing a remedy for treating an immune-mediated disorder in a subject. Preferably, the subject is a human or non-human vertebrate.

In a more preferable embodiment, the treatment of immune-mediated disorder is carried out by a mechanism selected from a group comprising inhibiting the proliferation of immune cells activated with Toll like receptor 9 agonist, inhibiting the activation of Toll like receptor 9, inhibiting interferon production and rescuing a subject from cytokine-mediated lethal shock.

Preferably, the immune-mediated disorder is systemic lupus erythematosus (SLE) which is treated by inhibiting TLR9 activation and interferon production induced by TLR9 agonists, viruses and the serum of SLE patient, the immune-mediated disorder is sepsis which is treated by rescuing a subject from cytokine-mediated lethal shock, or the immune-mediated disorder is multiple organ dysfunction syndromes which is treated by rescuing a subject from cytokine-mediated lethal shock.

In another aspect, the present invention provides a remedy for administrating to a subject having or at risk of developing the immune-mediated disorder comprising above oligonucleotide.

Preferably, the remedy further comprises a pharmaceutically acceptable carrier and/or additional active ingredients. And more preferably, the remedy is in a form for administrating through the route including the enteral, parenteral and topical administration or inhalation.

In a preferable embodiment, the oligonucleotide can be pegylated.

### BRIEF DESCRIPTION OF DRAWINGS

Figure 1 shows graphs depicting the inhibition of SAT05f on the proliferation of human PBMC stimulated by CpG 2006 and CpG C274.
Figure 2 shows a graph depicting the inhibition of SAT05f on interferon production from human PBMC stimulated by CpG C274.
Figure 3 shows graphs depicting the inhibition of SAT05f on interferon production from human PBMCs stimulated with herpes simplex virus-type 1 virus and flu virus
   (A) Comparison of the anti-viral activity between the interferon (IFN) induced by inactivated HSV-1 and recombinant human interferon (IFN)-α. 'IU' represents the international unit. (B) Inhibitory effect of SAT05F on IFN production from human PBMC (hPBMC) stimulated by inactivated HSV-1. (C) The dose effect of SAT05F on IFN production from human PBMCs stimulated by HSV-1. (D) Comparison of the anti-viral activity between the interferon (IFN) induced by inactivated PR8 and recombinant human interferon (IFN)-α. (E) Inhibitory effect of SAT05F on IFN production from hPBMC stimulated with inactivated PR8. (F) The dose effect of SAT05F on IFN production from hPBMC stimulated with inactivated PR8.
   Data from one representative experiment of three are shown. HSV-1 represents herpes simplex virus-type 1 virus. PR8 represents flu virus (H1N1/PR8).

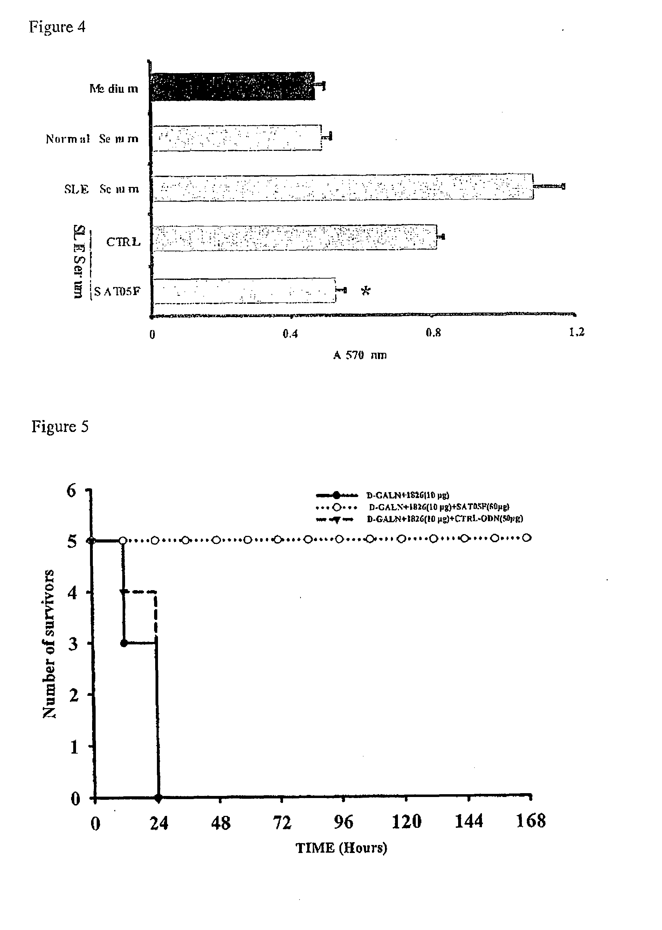

Figure 4 is a graph showing that SAT05F inhibits interferon production of from hPBMCs stimulated with serum of SLE patients.
Figure 5 is a graph showing that SAT05F rescues mice from cytokine-mediated lethal shock.

### EXAMPLES

The invention will now be described in more detail in the following Examples. But the invention is not limited to these Examples. In these Examples, herein, experiments using commercially available kits and reagents were done according to attached protocols, unless otherwise stated. The skilled artisan will appreciate that the oligonucleotides of the present invention can easily be applied to treat an immune-mediated disorder. The present invention will now be demonstrated by the following non-limiting examples.

All reagents used to manipulate the oligonucleotides (ODNs) in the following examples were pyrogen-free. The endotoxin in the ODN preparations was tested by using the Limulus amebocyte lysate assay (Associates of Cape Cod, Inc).Human PBMCs (hPBMC), used in the following samples, were isolated from buffy coats (The Blood Center of Jilin Province, China) by Ficoll-Hypaque (Pharmacia) density gradient centrifugation (P. M. Daftarian et al., (1996): Journal of Immunology, 157, 12-20). The cells were cultured in IMDM supplemented with 10% (v/v) heat-inactivated fetal bovine serum (FBS; GIBCO) and antibiotics (100 IU of penicillin/ml and 100 IU of streptomycin/ml) at 37°C in a 5% CO2 humidified incubator. The viability of the cells was 95-99% as determined by trypan blue exclusion.

### Example 1

### Effect of SAT05f on CpGODN induced proliferation of human PBMC

The oligonucleotides (ODNs) used in the example were synthesized in Sangon Biotech Company (Shanghai, China) and were CpG2006 (5'-tcgtcgttttgtcgttttgtcgtt-3'), CpG C274 (5'-tcgtcgaacgttcgagatgat 3'), A151 (5'-ttagggttagggttagggttaggg-3') (Hidekazu Shirota, et al. The Journal of Immunology, 2005, 174: 4579-4583), SAT05f (5'-cctcctcctcctcctcctcctcct-3') and Control ODN (5'-gttagagattaggca-3').

CpG2006 (Dominique De Wit, et al. Blood, 2004, Vol 103, Num 3:1030-103) is a prototype of B-type CpG ODN. CpG C274 (Omar Duramad, et al. The Journal of Immunology, 2005,174: 5193-5200) is a prototype of C type CpG ODN.

[³H] thymidine incorporation assay was used to test whether SAT05f inhibited the proliferation of hPBMC stimulated by CpG2006 or CpG C274. Briefly, hPBMCs (5×10⁵/well) were plated in 96-well U-bottomed plates (Costar) and cultured with CpG2006 (1 µg/ml) or CpG C274 (1 µg/ml) in the presence of SAT05f or A151 or Control ODN for 48 h, followed by pulsing with [³H] thymidine (New England Nuclear, Boston, MA) for 16 h. The cells were harvested on glass fiber filters and detected in a scintillation counter. The cell proliferation in triplet wells was expressed as mean cpm (counts per minute) ± SD.

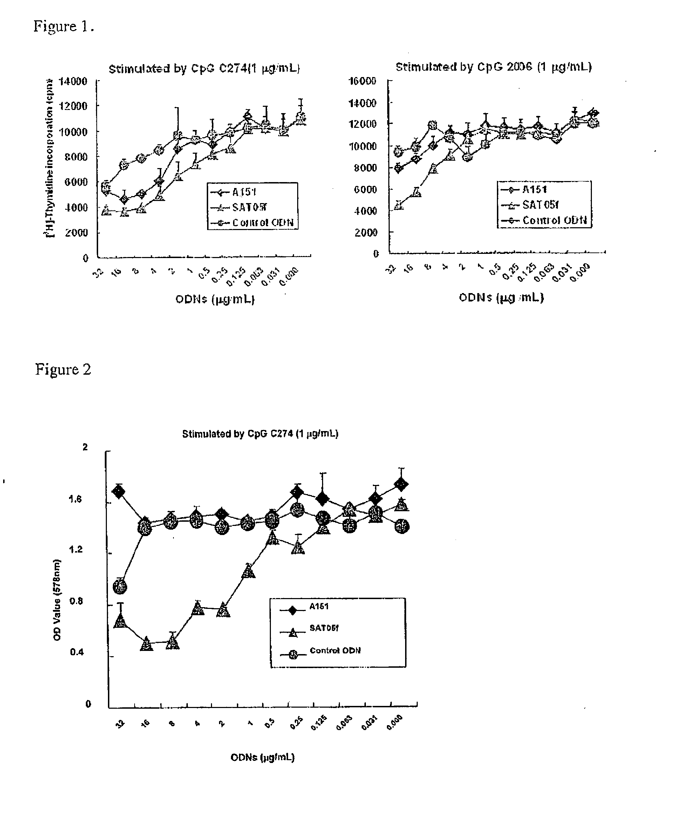

As shown in Figure 1, SAT05f inhibits the proliferation of hPBMCs stimulated with CpG ODN 2006 or CpG ODN C274. The inhibitory effect is stronger than that induced by A151. Control ODN can not induce the inhibition. Since CpG ODN including CpG2006 or CpG C274 is a TLR9 agonist [D.M. Klinman, Nat. Rev., Immunol. 4 (2004) 249-258], the data indicate that SAT05f inhibits TLR9 activation and can be used to treat the diseases related to TLR9 activation and other Toll-like receptor (TLR)-mediated diseases.

### Example 2

### Effect of SAT05f on CpGODN-induced interferon production from human PBMC

### <Experimental Method>

Vero E6 cells (African green monkey kidney cell line, American Type Culture Collection) were cultured at 37°C in a 5% CO2 humidified incubator and maintained in IMDM supplemented with 10% (v/v) heat-inactivated fetal bovine serum (FBS; GIBCO) and antibiotics (100 IU of penicillin/ml and 100 IU of streptomycin/ml).

An interferon (IFN) bioassay using Vero E6 cells and VSV was performed to test whether SAT05f inhibited the IFN production from hPBMC stimulated by CpG C274. The ODNs including CpG C274, A151, SAT05f and Control ODN were synthesized in Sangon Biotech Company (Shanghai, China). The sequences of CpG C274, A151, SAT05f and Control ODN are indicated as in example 1. CpG C274 is a prototype of C type CpG ODN and shares the activities of both A type CpG ODN and B type CpG ODN. A-type CpG ODN and capable of activating human plasmacytoid dendritic cells (pDCs) to produce large amount of type I interferon. hPBMCs (5×10⁵/well) were plated in 96-well U-bottomed plates (Costar) and cultured with CpG C274 (1 µg/ml) in the presence of SAT05f or A151 or Control ODN for 48 h and the supernatants were then collected for assaying their IFN activity. Vero E6 cells (3X10⁴/well) were seeded into 96-well flat-bottomed plates and cultured for 24 h to confluence. The cells were then incubated with 100 µl of the supernatants for 18 h and then challenged with 10X TCID50 (50% tissue culture infectious doses) of VSV for another 48 h. Vesicular stomatitis virus (VSV) was grown in Vero E6 cells. After titration, the virus was stored in aliquots at -70oC until use. After staining with 0.5% crystal violet, the cytopathic effect of virus was examined using Multi-well Microtiter Plate Reader at A578 nm and expressed as OD values.

### <Experimental Results>

As shown in Figure 2, SAT05f inhibits IFN production from hPBMC stimulated with CpG C274. Since elevated production of IFNs resulted from TLR-9 activation has been reported to contribute to the developing systemic lupus erythematosus (SLE) (Barrat FJ, et al. J Exp Med 2005; 202:1131-9; Wellmann U, et al. Proc Natl Acad Sci USA 2005; 102:9258-63), the data indicate that SAT05f can be used as a remedy to treat SLE and other Toll-like receptor (TLR)-mediated diseases by inhibiting elevated IFN production.

### Example 3. Inhibitory effect of SAT05F on interferon production from human PBMCs stimulated with herpes simplex virus-type 1 and flu virus

### <Experimental Method>

Vero E6 cells were cultured as described in example 2. HSV-1(herpes simplex virus-type 1) and PR8 (H1N1/PR8, a lab used flu virus) were originally obtained from Department of Immunology, Medical College of Norman Bethune, Jilin University, Changchun. HSV-1 (MOI=200) was propagated on Vero E6 cells and PR8 (MOI=3) was propagated on MDCK cells (Madin-Darby Canine Kidney Cells, ATCC). The MDCK cells were maintained in IMDM supplemented with 10% (v/v) heat-inactivated fetal bovine serum (FBS; GIBCO) and antibiotics (100 IU of penicillin/ml and 100 IU of streptomycin/ml). HSV-1 in IMDM supplemented with 2% (v/v) FBS was inactivated by heating at 70°C in a water bath for 10 min and PR8 in IMDM supplemented with 2% (v/v) FBS was inactivated by heating at 56°C in a water bath for 30 min.

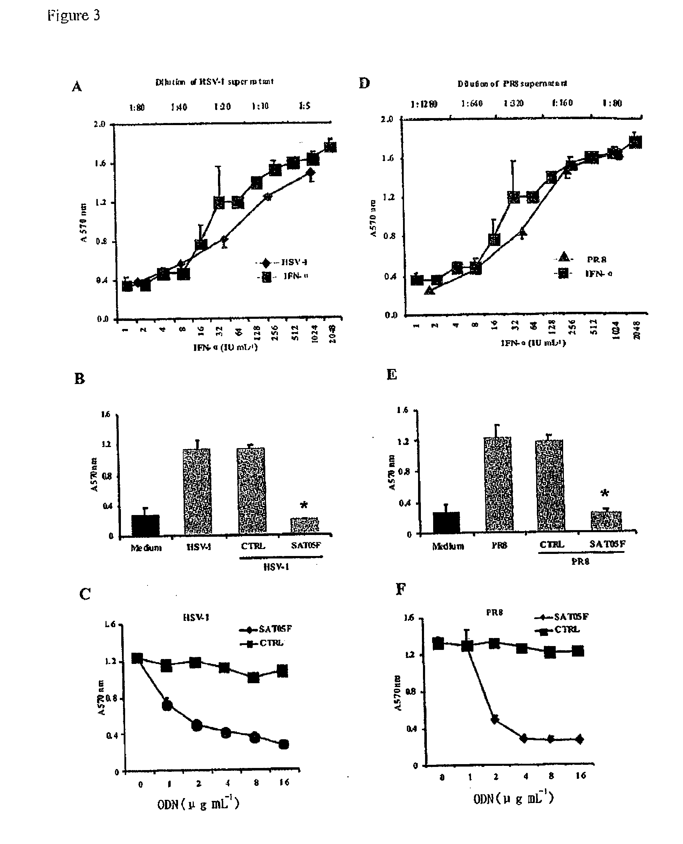

The ODNs including SAT05F and CTRLODN (indicated as CTRL in the Figure 3) with nucleotide sequence of 5'-aaaaataaaaataaaataaaat-3' were synthesized by Takara Co (Dalian, China). The sequence of SAT05F is indicated as in the example 1.

hPBMCs (5×10⁶/ml) were cultured in 96 well plate with inactivated HSV-1 or inactivated PR8 in the absence or presence of SAT05F or control ODN (CTRL ODN) at 37°C for 48 h. The supernatants were then collected for assaying. The Vero E6 cells were seeded into 96-well flat-bottomed plates (3X10⁴/well) and cultured for 24 h to confluence. The cells were incubated with 100 µl of the diluted supernatants (1:20 diluted for the HSV-1 induced and 1:80 diluted for the PR8 induced) for 16 h and then challenged with 10×TCID50 (50% tissue culture infectious doses) of VSV for another 48 h. After staining with 0.5% crystal violet, the cytopathic effects were examined using Multi-well Microtiter Plate Reader at A570 nm and expressed as mean OD value ± SD. Data from one representative experiment of three are shown.

### <Experimental Results>

As shown in Figure 3-A, inactivated HSV-1 virus could induced IFN that protected the Vero E6 cells from VSV attacking as recombinant human interferon IFN-αdid. As shown in Figure 3-B, SAT05F inhibited IFN production from human PBMC (hPBMC) stimulated by inactivated HSV-1 virus. Dose effect analysis revealed that SAT05F could significant IFN production from human PBMC stimulated with inactivated HSV-1 in a dose dependent way. SAT05F at 1µg/ml is efficient to mediate the inhibition (Figure 3-C). As shown in Figure 3-D, inactivated flu virus (PR8) could induced IFN efficient to protect the Vero E6 cells from VSV attacking as recombinant human interferon (IFN)-αdid. As shown in Figure 3-E, SAT05F inhibited interferon (IFN) production from human PBMC (hPBMC) stimulated by inactivated flu virus (PR8). Dose effect analysis revealed that SAT05F at 2µg/ml was efficient to inhibit the IFN production from hPBMC stimulated with inactivated PR8 and the inhibition reaches the maximum when SAT05F wass used at 4µg/ml (Figure 3-F).

It has been demonstrated that flu virus recognizes and activates TLR7 (Wang JP, et al. Blood. 2008 Jun 10. [Epub ahead of print]), and that HSV-1 recognizes and activates TLR9 (Hubertus Hochrein et al. PNAS, 101, 11416-11421), stimulating the production of IFN. Together with the results of the example, the oligonuleotides of the invention can be used as a remedy for the treatment of Toll-like receptor (TLR)-mediated disease such as SLE by inhibiting TLR7 or TLR9 activation and IFN production induced by virus.

### Example 4.

The inhibitory effect of SAT05F on interferon production of from hPBMCs stimulated with serum of SLE patients.

### <Experimental Method>

Anti-dsDNA-positive sera of SLE patients were obtained from Department of Rheumatology, China-Japan Union Hospital, Jilin University. ODNs including SAT05F and CTRL-ODN (indicated as CTRL in the Figure 4) with the sequence of 5'-AAAAATAAAAATAAAATAAAAT-3' were synthesized by Takara Co (Dalian, China). The sequence of SAT05F is indicated as in example 1.

hPBMCs (5×10⁶/ml) were cultured in 96 well plate with 1:1 diluted anti-dsDNA-positive serum of SLE patient in the absence or presence of SAT05F or CTRL ODN for 48 h. hPBMCs incubated with medium alone or with anti-dsDNA-negative serum of healthy blood donor were set as controls. The supernatants were collected for assaying their IFN activity. The Vero cells were incubated with the collected supernatant (1:5 diluted) for 16 h. and then attacked by 10TCID50 of VSV for 48 h. After staining with 0.5% crystal violet, the cytopathic effects were examined using a multi-well microtiter plate reader at A570 nm.

### <Experimental Results>

As indicated in Figure 4, the serum from SLE patient stimulates IFN production from hPBMC, and SAT05F inhibits the IFN production from human PBMC stimulated with the serum from SLE patient. Data from one representative experiment of three are shown. It is well established that elevated production of IFNs contributes to the development of SLE (Barrat FJ, et al. J Exp Med 2005; 202:1131-9; Wellmann U, et al. Proc Natl Acad Sci USA 2005; 102:9258-63). It has been demonstrated that endogenous IFN inducing factors has been reported existed in the serum of SLE patient (Kwok SK, et al. Arthritis Res Ther. 2008;10(2):R29), SLE patients have a circulating inducer of IFN production, sera from SLE patient frequently induce the production of IFN in cultures of PBMC from healthy blood donors (Vallin H, et al Clin Exp Immunol. 1999 Jan;115(1):196-202), and that anti-double-stranded DNA antibodies or DNA-anti-DNA Ab complexes functions as endogenous IFN-alpha inducers in SLE patient and contribute to the pathogenesis of SLE (Vallin H, et al. J Immunol. 1999 Dec 1;163(11):6306-13). Together, the data indicate that SAT05F can be used for the treatment of SLE patients by inhibiting IFN production,

### Example 5

### The effect of SAT05F on rescuing mice from cytokine-mediated lethal shock

### <Experimental Method>

In order to elucidate the in vivo functions of SAT05F, a model of cytokine-mediated lethal shock was induced with the reference of a general method (Peter M, et al. Immunology. 2008 Jan;123(1):118-28; Marshall AJ, et al. Infect Immun. 1998 Apr; 66(4):1325-33)..

Female BALB/C mice (20±1 g weight) obtained from the Experimental Animal Center, Medical College of Norman Bethune, Jilin University) were given free access to food and water during the experiment. The experiments were in accordance with local legislation.

Oligonucleotides including SAT05F with the sequence of 5'-cctcctcctcctcctcctcctcct-3', CTRL-ODN with the sequence of 5'-aaaaataaaaataaaataaaat-3' and CpG-ODN 1826 (1826) with the sequence of 5'-tccatgacgttcctgacgtt-3'[Sanjai Kumar, et al. Infection and Immunity, February 2004, p. 949-957, Vol. 72, No. 2] were synthesized by Takara Co (Dalian, China).

D-galactosamin (D-(+)-Galactosamine HCL, D-GALN) was from DeBioCbem, Nanjing, China.

The BALB/C mice, five in each group, were divided into groups of D-GALN+1826, D-GALN+1826+SAT05F, D-GALN+1826+CTRL-ODN. The mice were injected intraperitoneally (i.p.) with 500µl of D-galactosamin (32 mg/ml in PBS). 1.5h later, the mice were intraperitoneally (i.p.) injected with 1826 (10µg/ per mouse in PBS) and subsequently injected (i.p.) with 50µg of SAT05F (in PBS) (in D-GALN+1826+SAT05F group) or CTRL-ODN (in D-GALN+1826+CTRL-ODN group). In D-GALN+1826 group, the mice were injected with D-galactosamin and 1826 only. The mice were monitored and lethality was recorded.

### <Experimental Results>

As indicated in Figure 5 , in the D-GALN+1826 group or D-GALN+1826+CTRL-ODN group of the model, all of the five mice were deprived of life in 24 after the D-galactosamin injection, Comparatively, in 168 hours after the D-galactosamin injection, all of five mice in D-GALN+1826+SAT05F group survived, demonstrating that SAT05F can rescue the mice received D-galactosamin and 1826. It was documented that D-galactosamin presensitized mice could be created into cytokine-mediated lethal shock animal models by injecting CpG OD (Peter M, et al. Immunology. 2008 Jan;123(1):118-28). As apparently shown in these results, the data indicate that SAT05F is suppressive in vivo and inhibits cytokine-mediated lethal shock. Data from one representative experiment of two are shown.

## Claims

1. Use of an oligonucleotide for preparing a remedy for treating an immune-mediated disorder in a subject, wherein
the oligonucleotide includes a sequence that fits the formula of (5' CCT 3') n, wherein 5' CCT 3' is a repeat unit and n is an integer from 2 to 50 and the immune-mediated disorder is systemic lupus erythematosus, sepsis, multiple organ dysfunction syndromes or psoriasis.

2. The use of claim 1, wherein the subject is a human or non-human vertebrate.

3. The use of claim 1, wherein the treatment of immune-mediated disorder is carried out by a mechanism selected from a group comprising inhibiting the proliferation of immune cells activated with Toll like receptor 9 agonist, inhibiting the activation of Toll like receptor 9, inhibiting interferon production and rescuing a subject from cytokine-mediated lethal shock.

4. The use of claim 1, wherein the immune-mediated disorder is systemic lupus erythematosus (SLE) which is treated by inhibiting TLR9 activation and interferon production induced by TLR9 agonists, viruses and the serum of SLE patient, the immune-mediated disorder is sepsis which is treated by rescuing a subject from cytokine-mediated lethal shock, or the immune-mediated disorder is multiple organ dysfunction syndromes which is treated by rescuing a subject from cytokine-mediated lethal shock.

5. An oligonucleotide including a sequence that fits the formula of (5'CCT 3') n, for use in the treatment of a subject having or at risk of developing the immune-mediated disorder,
wherein
5' CCT 3' is a repeat unit and n is an integer from 2 to 50 and the immune-mediated disorder is systemic lupus erythematosus, sepsis, multiple organ dysfunction syndromes or psoriasis.

6. The oligonucleotide for use according to claim 5, wherein the oligonucleotide is administered with a pharmaceutically acceptable carrier and/or additional active ingredients.

7. The oligonucleotide for use according to claim 6, wherein the oligonucleotide can be pegylated.

8. The oligonucleotide for use according to claim 7, wherein the oligonucleotide is in a form for administrating through the route including the enteral, parenteral and topical administration or inhalation.

## Patentansprüche

1. Verwendung eines Oligonukleotids zur Herstellung eines Heilmittels für die Behandlung einer immunvermittelten Erkrankung in einem Patienten, wobei
das Oligonukleotid eine Sequenz enthält, die zu der Formel (5' CCT 3') n passt, wobei 5' CCT 3' eine Wiederholungseinheit ist und n eine ganze Zahl von 2 bis 50 ist und die immunvermittelte Erkrankung systemischer Lupus erythematodes, Sepsis, multiple Organdysfunktionssyndrome oder Psoriasis ist.

2. Verwendung nach Anspruch 1, wobei der Patient ein menschliches oder nichtmenschliches Wirbeltier ist.

3. Verwendung nach Anspruch 1, wobei die Behandlung der immunvermittelten Erkrankung durch einen Mechanismus ausgeführt wird, der aus folgender Gruppe ausgewählt ist: Hemmen der Proliferation von Immunzellen, die mit Toll-ähnlichem Rezeptor-9-Agonist aktiviert wurden, Hemmen der Aktivierung von Toll-ähnlichem Rezeptor 9, Hemmen der Interferonproduktion und Retten eines Patienten vor einem cytokinvermittelten letalen Schock.

4. Verwendung nach Anspruch 1, wobei die immunvermittelte Erkrankung systemischer *Lupus erythematodes* (SLE) ist, der durch Hemmen der TLR9-Aktivierung und Interferonproduktion, die durch TLR9-Agonisten, Viren und das Serum von SLE-Patienten induziert wird, behandelt wird, die immunvermittelte Erkrankung Sepsis ist, die durch Retten eines Patienten vor einem cytokinvermittelten letalen Schock behandelt wird, oder die immunvermittelte Erkrankung multiple Organdysfunktionssyndrome sind, die durch Retten eines Patienten vor einem cytokinvermittelten letalen Schock behandelt werden.

5. Oligonukleotid, das eine Sequenz enthält, die zu der Formel (5' CCT 3') n passt, zur Verwendung bei der Behandlung eines Patienten mit einer immunvermittelten Erkrankung oder dem Risiko der Entstehung der immunvermittelten Erkrankung, wobei 5' CCT 3' eine Wiederholungseinheit und n eine ganze Zahl von 2 bis 50 ist und die immunvermittelte Erkrankung systemischer Lupus erythematodes, Sepsis, multiple Organdysfunktionssyndrome oder Psoriasis ist.

6. Oligonukleotid zur Verwendung nach Anspruch 5, wobei das Oligonukleotid mit einem pharmazeutisch unbedenklichen Träger und/oder zusätzlichen Wirkstoffbestandteilen verabreicht wird.

7. Oligonukleotid zur Verwendung nach Anspruch 6, wobei das Oligonukleotid pegyliert sein kann.

8. Oligonukleotid zur Verwendung nach Anspruch 7, wobei das Oligonukleotid in einer Form zum Verabreichen auf enteralem, parenteralem und topischem Weg durch Verabreichung oder Inhalation vorliegt.

## Revendications

1. Utilisation d'un oligonucléotide pour préparer un remède pour traiter un trouble à médiation immune chez un sujet, dans lequel
l'oligonucléotide comprend une séquence qui s'ajuste à la formule de (5' CCT 3')n, dans laquelle 5' CCT 3' est un motif répété et n est un nombre entier de 2 à 50 et le trouble à médiation immune est le lupus érythémateux systémique, la sepsie, les syndromes de dysfonctionnement d'organes multiples ou le psoriasis.

2. Utilisation selon la revendication 1, dans laquelle le sujet est un humain ou un vertébré non humain.

3. Utilisation selon la revendication 1, dans laquelle le traitement du trouble à médiation immune est réalisé par un mécanisme choisi dans un groupe comprenant l'inhibition de la prolifération de cellules immunitaires activée avec l'agoniste du récepteur 9 de type Toll, l'inhibition de l'activation du récepteur 9 de type Toll, l'inhibition de la production d'interféron et le sauvetage d'un patient d'un choc létal médié par cytokine.

4. Utilisation selon la revendication 1, dans laquelle le trouble à médiation immune est le lupus érythémateux systémique (LES) qui est traité en inhibant l'activation de TLR9 et la production d'interféron induite par les agonistes de TLR9, les virus et le sérum du patient LES, le trouble à médiation immune est la sepsie qui est traitée en sauvant un sujet du choc létal médié par cytokine ou le trouble à médiation immune est les syndromes de dysfonctionnement d'organes multiples qui sont traités en sauvant un sujet du choc létal médié par cytokine.

5. Oligonucléotide comprenant une séquence qui s'ajuste à la formule de (5' CCT 3')n, pour une utilisation dans le traitement d'un sujet ayant ou à risque de développer le trouble à médiation immune,
dans lequel
5' CCT 3' est un motif répété et n est un nombre entier de 2 à 50 et le trouble à médiation immune est le lupus érythémateux systémique, la sepsie, les syndromes de dysfonctionnement d'organes multiples ou le psoriasis.

6. Oligonucléotide pour une utilisation selon la revendication 5, dans lequel l'oligonucléotide est administré avec un véhicule pharmaceutiquement acceptable et/ou des principes actifs supplémentaires.

7. Oligonucléotide pour une utilisation selon la revendication 6, dans lequel l'oligonucléotide peut être pégylé.

8. Oligonucléotide pour une utilisation selon la revendication 7, dans lequel l'oligonucléotide est sous une forme pour une administration par la voie comprenant l'administration entérale, parentérale et topique ou par inhalation.


### SEQUENCE LISTING

<110> Changchun Huapu Biotechnology Co., Ltd.
   <120> Oligonucleotide and use thereof
   <130>
   <160> 6
   <170> PatentIn version 3.1
<210> 1
   <211> 24
   <212> DNA
   <213> Artificial Sequence
<400> 1
   cctcctcctc ctcctcctcc tcct 24
<210> 2
   <211> 24
   <212> DNA
   <213> Artificial Sequence
<400> 2
   tcgtcgtttt gtcgttttgt cgtt 24
<210> 3
   <211> 21
   <212> DNA
   <213> Artificial Sequence
<400> 3
   tcgtcgaacg ttcgagatga t 21
<210> 4
   <211> 24
   <212> DNA
   <213> Artificial Sequence
<400> 4
   ttagggttag ggttagggtt aggg 24
<210> 5
   <211> 15
   <212> DNA
   <213> Artificial Sequence
<400> 5
   gttagagatt aggca 15
<210> 6
   <211> 22
   <212> DNA
   <213> Artificial Sequence
<400> 6
   aaaaataaaa ataaaataaa at 22